(19) **Europäisches Patentamt / European Patent Office / Office européen des brevets**

(11) **EP 2 910 242 B1**

(12) **EUROPEAN PATENT SPECIFICATION**

(45) Date of publication and mention
of the grant of the patent:
**10.04.2019 Bulletin 2019/15**

(21) Application number: **12886774.4**

(22) Date of filing: **19.10.2012**

(51) Int Cl.:
*A61K 9/22* [(2006.01)]          *A61K 9/52* [(2006.01)]
*A61K 31/485* [(2006.01)]       *A61P 25/04* [(2006.01)]
*A61K 9/00* [(2006.01)]          *A61K 9/16* [(2006.01)]
*A61K 9/50* [(2006.01)]

(86) International application number:
**PCT/CN2012/001404**

(87) International publication number:
**WO 2014/059558 (24.04.2014 Gazette 2014/17)**

(54) **DURABLE ANALGETIC SEBACOYL DINALBUPHINE ESTER-PLGA CONTROLLED RELEASE FORMULATION**

DAUERHAFTE ANALGETISCHE SEBACOYL-DINALBUPHIN-ESTER-PLGA-FORMULIERUNG MIT GESTEUERTER FREISETZUNG

FORMULATION À LIBÉRATION CONTRÔLÉE D'ESTER DE DINALBUPHINE DE SÉBACOYLE-PLGA ANALGÉSIQUE DURABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.08.2015 Bulletin 2015/35**

(73) Proprietor: **Hu, Oliver Yaopu**
**Tingzhou Rd., Taipei, Taiwan 100 (CN)**

(72) Inventors:
• **HU, Oliver Yaopu**
**Taipei**
**Taiwan 100 (CN)**
• **HU, Yufang**
**Taipei**
**Taiwan 100 (CN)**
• **CHANG, Chenchung**
**Taipei**
**Taiwan 100 (CN)**

(74) Representative: **Cabinet Chaillot**
**16/20, avenue de l'Agent Sarre**
**B.P. 74**
**92703 Colombes Cedex (FR)**

(56) References cited:
**WO-A1-2011/009602      CN-A- 1 321 643**
**CN-A- 102 573 805        US-A1- 2004 024 006**

• **FANG-I. LIU ET AL.: 'Biodegradable polymeric microspheres for nalbuphine prodrug controlled ielivery: in vitro characterization and in vivo pharmacokinetic studies.' INTERNATIONAL JOURNAL OF PHARMACEUTICS. vol. 257, 2003, pages 23 - 31, XP055255665**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND OF THE INVENTION**

**1. FIELD OF THE INVENTION**

The present invention features a formulation comprising of sebacoyl dinalbuphine ester and a common pharmaceutical and biodegradable excipient PLGA polymer for treating acute or chronic pain in mammalians, and said formulation is a long-term controlled release formulation which can be easily absorbed in the body with the help from polymer molecules and thus maintain effective serum concentration of nalbuphine, which consequently improved the dosage and frequency of the traditionally used nalbuphine injections .

**2. DESCRIPTION OF THE PRIOR ART**

Pain is a sensation caused by stimulation of nociceptors in peripheral nerve endings, and is usually triggered by either external mechanical, thermal, or chemical stimuli, or by internal chemical or electrical stimuli. The stimulation through chemical or nociceptive pathway activates the cerebral cortex or central nervous system and results in the feeling of pain. Nearly all mammalians and even some invertebrates such as squid can perceive pain, and have developed corresponding responses including withdraw or stay away from the source to cope with the pain. If not controlled or relieved, pain will induce considerable stress and result in the increase of ACTH, cortisol, ADH, catecholamine, and glucose as well as decrease of insulin and testosterone in serum, thereby affecting numerous physiological functions including cardiovascular, respiratory, and metabolic functions, even tissue healing. The pain response is part of a protective reflex system to alert the body of severe situations and tissue damage. Hence, for the patients with constant pain, long-term analgesics are needed, and the long-lasting effect is crucial for patients with acute or chronic pain. The pain may last a few days to several months. For example, acute pain, such as post-surgical pain, pain induced by trauma or burn, may last 4 to 6 days; chronic pain including non-malignant and cancer pain, on the other hand, may persist from weeks to several months. One significant breakthrough in the history of fighting pain is the purification of aspirin and morphine by scientists in the late 19th century. Both drugs are well-documented and are effective analgesics. Yet, the analgesic effect of aspirin is not only mild, but it may cause mucosal damage in stomach, inhibition of platelet aggregation, and even stomach bleeding. Moreover, though morphine exhibits superior analgesic effects, its side effects further restrict the possible applications. Therefore, use with caution and under careful supervision is necessary when treating with these drugs. The new drugs developed in the twentieth century are nonetheless within the scope of non-steroidal anti-inflammatory drugs (for anti-inflammatory and pain relief) and morphine (for morphine receptors in the central nervous system).

At present, the available morphine-like analgesics on the market only act for a limited time and may produce serious health effects; those drugs include sufentanil, remifentanil, fentanyl, and morphine injections. In addition, two to three injections a day is required for long-term treatment, which is inconvenient, and overdose may result in respiratory inhibition and addiction. Therefore, development of an analgesic that has lasting effect and mild side effects is the focus of the present invention. Nalbuphine is a strong analgesic that can be used for treating patients with medium to severe pain. Results from treating post-surgical pain with nalbuphine and morphine have shown that both drugs are equally effective; yet, nalbuphine induces fewer side effects with less respiratory inhibition and addiction. Pharmacologically, Nalbuphine is an opioid receptor kappa agonist, also a partial Mu-receptor antagonist (*Schmidt W.K. et al.*, *1985*); thereby the ceiling effect caused by this mechanism will not give rise to further respiratory inhibition if the amount is over 30 mg. In order to come up to the top ten countries that have the most advanced drug/medicine manufactures, the Executive Yuan issued an announcement, No. 0960004264, stating that nalbuphine is not a controlled drug. The pain-relieving effect of nalbuphine lasts from 3 hrs (serum concentration ~10 ng/ml) to 6 hrs (~2.5 ng/ml). Patients who are treated with this drug have to be admitted to the hospital and received multiple injections, which is not only a waste of medical resources, but a pain for the patients. The prosoft-drug design can improve the half life of drugs and has been widely used in clinical practice with superior results. Nalbuphine is more a hydrophilic drug when compared with a prosoft-drug. Esterification of nalbuphine using long chain carbonic acid increases the lipid solubility of the prodrug, which further allows embedding of the drug with oily or biodegradable substances and prolongs the effects by controlled release of the drug in tissues upon intramuscular injection. Additionally, esterase is found in various tissues and organs such as blood, brain, liver, heart, lung, kidney, and muscle, and the pharmacological effects and safety of nalbuphine prosoft-drug were reported exactly the same as of the prodrug.

SDE is one prosoft-drug of nalbuphine which can be used to effectively treat acute, chronic, and post-surgical pain. In present invention, nalbuphine prosoft-drug SDE and PLA, a biocompatible and biodegradable polymer, were combined and used to produce a long-term controlled-release formulation form. The purpose of producing drugs with long-term efficacy and controlled release is to prolong the effective time of a single dose treatment, which requires fewer dosage, reduces the possibility of missing a dose, and helps to maintain a steady serum concentration of the drugs and improve the efficacy. Patients who require long-term use of analgesics usually hope they can be discharged from the hospitals and return to home or loved ones. Hence, after years of meticulous research, the inventors hereby reported a novel method for sebacoyl dinalbuphine ester synthesis with its chemical structure in R.O.C. patent no. 085106156 (nalbuphine ester). The present invention is founded on the prior invention (R.O.C. patent no. 089109293, a new formulation containing nalbuphine dinalbuphine ester prodrug for oral administration) that traditional methods cannot prolong the effect duration of nalbuphine particularly. The inventors successfully developed a controlled-release formulation form as described in the present invention, which comprises of the pro-soft drug sebacoyl dinalbuphine ester and a pharmaceutically acceptable and biodegradable PLGA polymer with prolonged efficacy in vivo, and a single dose of administration every two weeks or even every few months is sufficient for treatment. Furthermore, the inventors have published the discovery in several well-recognized international journals including electrically assisted method of transdermal delivery of other long-lasting and controlled released nalbuphine derivatives (*Jeng-Fen Huang et al.,2005*), plant oil injections(*Pao L.H. et al.,2005*) and other nalbuphine pro-soft drugs such as nalbuphine decanoate, nalbuphine enanthate, nalbuphine pivalate, nalbuphine propionate as well as implants mentioned in the present invention using pharmaceutically acceptable and biodegradable PLGA polymer formulation (*Sung K.C. et al.,1998*), and controlled-release of microspheres (*Fang-I. Liu et al., 2003*). However, the aims of prolonged duration of drugs or reduced dosage have not been accomplished. As mentioned previously, subcutaneous injection of PLGA microspheres containing nalbuphine, nalbuphine propionate, nalbuphine pivalate, or nalbuphine decanoate ((50 mg/rabbit; three mice each group) have shown that the effective serum concentration lasted only 4 days which is significantly shorter than expected (*Fang-I. Liu et al., 2003*) and may due to the insolubility in lipid of these pro-soft drugs. Nevertheless, later studies from the inventors suggested that injection of the long-term controlled release sebacoyl dinalbuphine ester-PLGA formulation with better lipid solubility (150 mg/kg of SDE, in rats, n=7) can regulate drug concentration and maintain effective concentration at 2.5 ng/ml for up to two weeks or even several months by taking the advantages of the PLGA polymer, e.g. PLA/PGA ratio and average molecular weight, etc. (as shown in Table 1). Hence, based on the abovementioned results, the finding of a formulation comprising of sebacoyl dinalbuphine ester (a pro-soft drug) and a biodegradable polymer PLGA (commonly used pharmaceutical excipient) for treating acute or chronic pain in mammalians is novel and inventive.

## SUMMARY OF THE INVENTION

The purpose of the present invention is to provide a long-term controlled release formulation form of nalbuphine that can solve the traditional efficacy duration problems and substantially prolong the effects of nalbuphine so as to improve its efficacy when compared with traditional single dose treatment. The advantages of this formulation is to reduce the dosage, avoid patients miss a dose, and improve efficacy and maintain stable serum levels after administration.

To achieve the forgoing aims, the present invention provides a pro-soft drug, a long-term controlled release formulation form of sebacoyl dinalbuphine ester. The use of PLGA which is a common pharmaceutical and biodegradable excipient in the formulation notably reduces traditional nalbuphine injection dosage. Moreover, the present invention also provides a proper long-term controlled release formulation form of the pro-soft drug, sebacoyl dinalbuphine ester, a common pharmaceutical and biodegradable PLGA excipient formulation wherein the PLGA excipient includes at least one relevant derivative of polylactic acid (PLA), polylactic glycolic acid (PGA), polylactic acid (PLA), poly glycolic acid (PGA), or their combinations thereof. Related derivatives of polylactic acid (PLA) and polylactic glycolic acid (PGA) include poly butylene succinate (PBS), polyhydroxyalkanoate (PHA), polycaprolactone acid lactone (PCL), polyhydroxybutyrate (PHB), glycolic amyl (PHV), PHB and PHV copolymer (PHBV), and poly lactic acid (PLA) - polyethylene glycol (PEG) copolymers (PLEG). The PLGA polymer formulation form can regulate the drug release rate via the characteristics of the polymer (e.g. PLA/PGA ratio and average molecular weight, etc.) and be used as analgesics. As for improvements of traditional nalbuphine injections, the results indicated that administration of long-term controlled release form of sebacoyl dinalbuphine ester, SDE-PLGA, (150 mg/kg of SDE) in SD rats can maintain the effective concentration at 2.5 ng/ml for two 2 weeks or even several months, and the serum concentration of the drug is regulated by different PLGA excipient combinations. For example, given PLGA at the ratio of 50:50 5k (n=5) can maintain the effective drug concentration at 2.5 ng/ml for around 21 days, whereas administration of PLGA (75:25 10k) (n=7) can uphold the same concentration for nearly 30 days. Lipid soluble drugs that contain long-term controlled release PLGA have higher matrix molecular weight and those drugs containing high percentage of PLA have lower release rate (PLA/PGA ratio and the range of molecular weights are PLA 50 ~100 %/PGA 0~50% and 5 k ~ 20 K, respectively). The in vivo experiments also demonstrated that combination of the pro-soft drug (sebacoyl dinalbuphine ester) and the common pharmaceutical and biodegradable PLGA polymer as excipient can significantly reduce the dosage of traditional nalbuphine injections. Therefore, the formulation is novel and inventive. The purpose of the present invention is to develop a long-term controlled release formulation form that can achieve the abovementioned aims. This new formulation of sebacoyl dinalbuphine ester developed in the present invention may include a pharmaceutically acceptable excipient such as diluents, fillers, binders, disintegrating agents, or lubricants. Moreover, the long-term controlled release formulation can be prepared in one of the following forms: tablets, capsules, soft capsules, pills, suspensions, microspheres, oral implants, emulsion injection, implantation agent and other pharmaceutically acceptable long-term controlled release formulation forms.

## BRIEF DESCRIPTION OF THE DRAWINGS

The foregoing summary, as well as the following detailed description of the invention, will be better understood when read in conjunction with the appended drawings. It should be understood, however, that the invention is not limited to the preferred embodiments shown.

Figure 1. Example: microsphere- long-term controlled release form.
Figure 2. Dissolution of the long-term controlled release formulation form in test group (75/25 10k), (75/25 15k), (75/25 18k), and (75/25 20k) by comparison of release of drugs with different molecular weights (mean±SD, n=3).
Figure 3. Dissolution of the long-term controlled release formulation form in control group (75/25 10k) and (100/0 10k) by comparison of release of the drugs containing various amount of PLA (mean±SD, n=3).
Figure 4. Dissolution of the long-term controlled release form in test group (50/50 5k) and (75/25 10k) by comparison of release of the drugs containing various concentrations of PLA and with different molecular weight (mean±SD, n=3).
Figure 5. In vivo nalbuphine-time profile after intramuscular injection of the long-term controlled release formulation form in SD-rat at 50:50 5k (150 mg/kg SDE), 75:25 10k (150 mg/kg SDE), and 75:25 18k (150 mg/kg SDE). (A) Cartesian coordinates. (B) Semi logarithmic coordinates.

## DETAILED DESCRIPTION OF THE PREFERRED EMBODIMENT

The present invention will now be described more specifically with reference to the following embodiments, which are provided for the purpose of demonstration rather than limitation.

### Example 1 Preparation of the long-term controlled release of SDE- PLGA formulation form

Experimental design and description:

### 1. Formulation of the long-term controlled release SDE- PLGA :

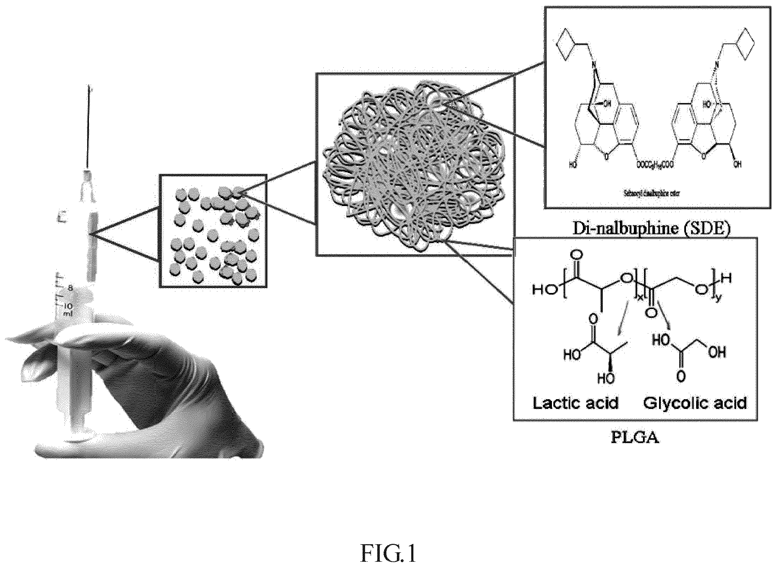

microsphere was used here as an example (Figure 1.)

Active substance

Sebacoyl dinalbuphine ester (SDE) is a pro-soft drug of nalbuphine. The pro-soft drug design can improve the half life of SDE which has become a common and effective drug used clinically.

Excipients

The excipient used herein is made of a combination of polylactide (PLA) and polyglycolide (PGA) in various ratios with superior biodegradability and biocompatibility.

i. A fixed amount of polymer PLGA (900 mg) and SDE powder (1,200 mg) were added to a clean bottle, followed by 9 ml organic solvent Dichloromethane (with stirring) to prepare the oil phase.
ii. The water phase consisted of 900 ml 0.5 % PVA, and was injected into the thermostat encapsulation reactor (the temperature was maintained at 8-10 , high temperature may cause degradation and production of holes) and was then mixed in a homogenizer at 1,800 RPM.
iii. An airtight needle was used for extraction of the oily phase and then injected into the water phase at the same speed to form the microspheres by sheer stress (homogenous time is 4 min). After stirring at 20°C for 2 hr (at the speed 300 RPM) for solidification, the microspheres were further stirred for 2 hr to eliminate organic solvent at the speed of 300 RPM.
iv. Filtration (upper filter 150 $\mu$m, lower filter 35 $\mu$m) to remove water phase and freeze-dry (lyophilized condition: -40.0°C, $1.0 \times 10^{-1}$ psi) to produce SDE-PLGA microspheres.

Formulation example 1

| PLGA polymer | 900 mg |
|---|---|
| SDE powder | 1200 mg |
| Total | 2100 mg |

Formulation example 2 (The molecular weight of PLGA is 5kDa)

| PLA polymer (50%) | 150 mg |
|---|---|
| PGA polymer (50%) | 150 mg |
| SDE powder | 400 mg |
| Total | 700 mg |

Formulation example 3 (The molecular weight of PLGA is 10kDa)

| PLA polymer (75%) : | 225 mg |
|---|---|
| PGA polymer (25%) : | 75 mg |
| SDE powder | 400 mg |
| Total | 700 mg |

Formulation example 4 (The molecular weight of PLGA is 15kDa)

| PLA polymer (75%) : | 225 mg |
|---|---|
| PGA polymer (25%) : | 75 mg |
| SDE powder | 400 mg |
| Total | 700 mg |

Formulation example 5 (The molecular weight of PLGA is 18kDa)

| PLA polymer (75%) : | 225 mg |
|---|---|
| PGA polymer (25%) : | 75 mg |
| SDE powder | 400 mg |
| Total | 700 mg |

Formulation example 6 (The molecular weight of PLGA is 20kDa)

| PLA polymer (75%) : | 225 mg |
|---|---|
| PGA polymer (25%) : | 75 mg |
| SDE powder | 400 mg |
| Total | 700 mg |

Formulation example 7 (The molecular weight of PLGA is 10kDa)

|  |  |
|---|---|
| PLA polymer (100%) : | 300 mg |
| PGA polymer (0%) : | 0 mg |
| SDE powder | 400 mg |
| Total | 700 mg |

**2. Determination of drug loading (%) and encapsulation (%) of SDE-PLGA drug using microsphere as the example:**

i. SDE-PLGA microsphere (>10 mg) were dissolved in acetonitrile and obtained mg microsphere/ ml acetonitrile solution (e.g. 11.2 mg SDE-PLGA microsphere in 11.2 ml acetonitrile) N=3。

ii. The obtained solution was the diluted in acetonitrile 100 times and $100\mu l$ was injected into HPLC system for calculation and determination of the loading percentage.

iii. Drug loading (%) = (API/(API+PLGA))*100 %

iv. Encapsulation (%)=(practical encapsulation/theoretical encapsulation)*100 %

**3. Determination of microsphere diameter by Laser :**

i. Adequate amount of microspheres were dissolved in the water and then added dropwise into the sample tank with vigorous stirring to ensure homogenous distribution.
ii. The sample tank was transferred to a infrared light scattering analyzer and the diameter of the microsphere was determined with a He-Ne laser (633 nm).
iii. The mean and standard deviation were calculated using the data collected from three independent experiments.

**Experimental results:**

Six biodegradable PLG polymer excipients (50:50 5 k, 75:25 10 k, 75:25 15 k, 75:25 18 k, 75:25 20 k, and 100:0 10k) were used for studying drug encapsulation, successful encapsulation rate, and average microsphere diameter variation at the same homogeneous speed (1800 RPM) and the same API/polymer ratio (400 mg/300 mg). The results indicated that no significant differences was found in either drug encapsulation (45-60 %) or average diameter differences (60-70 $\mu$m) at the same speed and with the same API/polymer ratio (400 mg/300 mg) (Table 2).

**Table 1 The molecular weight, oil-in-water coefficients (logP), and drug saturated solubility in water of nalbuphine and other pro-soft drugs.**

| Drug | Mw (Da) | logP | Aqueous solubility |
|---|---|---|---|
| Nalbuphine HCl | 357.46 | 0.17 | >25 mg/ml |
| Nalbuphine propionate | 413.43 | 1.05 | 44.67 ug/ml |
| Nalbuphine pivalate | 441.59 | 1.42 | 33.67 ug/ml |
| Nalbuphine enanthate | 469.62 | 1.94 | 3.00 ug/ml |
| Nalbuphine decanoate | 511.70 | 3.30 | 670 ng/ml |
| Sebacoyl dinalbuphine ester | 881.12 | 3.15 | <250 ng/ml |

**Table 2 Average particle size of drug-loaded microspheres, drug loading (%) and encapsulation efficiency (%) of various polymers.**

| Formulation | PLA/PGA ratio | Mw (Da) | API (mg) | PLGA (mg) | Homogenization speed (rpm) | Average particle size (μm) | Drug loading (%) | Encapsulation efficiency (%) |
|---|---|---|---|---|---|---|---|---|
| 1 | (50:50) | 5k | 400 | 300 | 1,800 | 59.53 ± 0.48 | 46.83 ± 2.35 | 81.95 ± 4.11 |
| 2 | (75:25) | 10k | 400 | 300 | 1,800 | 60.33 ± 0.17 | 57.48 ± 0.42 | 100.59 ± 0.73 |
| 3 | (75:25) | 15k | 400 | 300 | 1,800 | 69.68 ± 0.61 | 59.03 ± 1.27 | 103.30 ± 2.22 |
| 4 | (75:25) | 18k | 400 | 300 | 1,800 | 62.09 ± 0.13 | 54.84 ± 2.03 | 95.97 ± 3.57 |
| 5 | (75:25) | 20k | 400 | 300 | 1,800 | 62.28 ± 0.92 | 60.68 ± 3.18 | 106.19 ± 5.56 |
| 6 | (100:0) | 10k | 400 | 300 | 1,809 | 67.79 ± 0.10 | 48.15 ± 4.32 | 84.26 ± 7.57 |

**Example 2 In vitro dissolution test of long-term controlled release SDE- PLGA**

i. **In vitro dissolution testing- microspheres used here as an example:**
Screw-cap 16X125 mm test tubes were cleaned and sterilized, and 400 ml in vitro dissolution buffer containing 0.025 M PBS + 0.5 % Tween20 was then added to a 500 ml Erlenmeyer flask containing a stir bar and then sterilized.
ii. Around 5 mg microsphere powder (unsterilized because the process may change the nature of microspheres) was added to 400 ml dissolution buffer in the hood and incubated in a shaking bath at 800 RPM to ensure even suspension of the microspheres in the buffer.
iii. Samples of 5 ml microsphere suspension were then transferred to 16X125 mm test tubes and labeled with time (n=3). The tubes were sealed with transparent tapes (to prevent the water in the water bath leaks into the tubes and to ensure the screw cap is secured.)
iv. The tubes were then incubated in a water bath at 37 with a shaking rate of 100 RPM till the reaction is finished.
v. This method mainly measures the amount of drug released from the microspheres. Samples of 4.5 ml supernatant from these tubes labeled at different times were transferred to fresh tubes containing 4.5 ml acetonitrile to destroy the microspheres, and incubated in a shaker for 10 min. Upon completion of degradation, collected samples were then filtered through a 0.45μm PVDF membrane, and 200 μl of the filtered supernatant was injected directly into the HPLC system for further analysis. The results were calculated using the following formula: drug

$$\text{release } (\%) = 100(\%)\text{- remaining drug amount } (\%) \circ$$

**2.** Chromatographic conditions for high performance liquid chromatography (HPLC) analysis:

In vitro analysis such as determination of drug loading and encapsulation of SDE-PLGA as well as in vitro dissolution test were used for precise quatitification of drug concentration.
HPLC mobile phase consists of 25% acetate buffer (5μmol/L) and 75% ACN with 0.07% (volume ratio V/V) and 0.1% Triethylamine, a Thermo Hypersil Betasil Silica column (150x4.6 mm, 3μm, 30°C) was utilized for separation. The UV wavelength and flow rate were 210 nm and 1.3ml/min, respectively, and the sample analyzing time was 7 min per sample.

**3. Preparation of drug loading curve**

The pro-soft drug, nalbuphine dinalbuphine ester, and its prodrug were mixed in ACN solution to make standard solutions at 250, 500, 1000, 1500, 2000, 3500, 5000, 7500, and 10000 (ng/ml).
After analysis with HPLC, the peak area and its corresponding concentrations obtained from graphs of nalbuphine dinalbuphine ester, and its prodrug were plot to prepare two verification curves for determination of accuracy and precision using standard deviation (SD), variance coefficient (CV) and error.

**Results:**

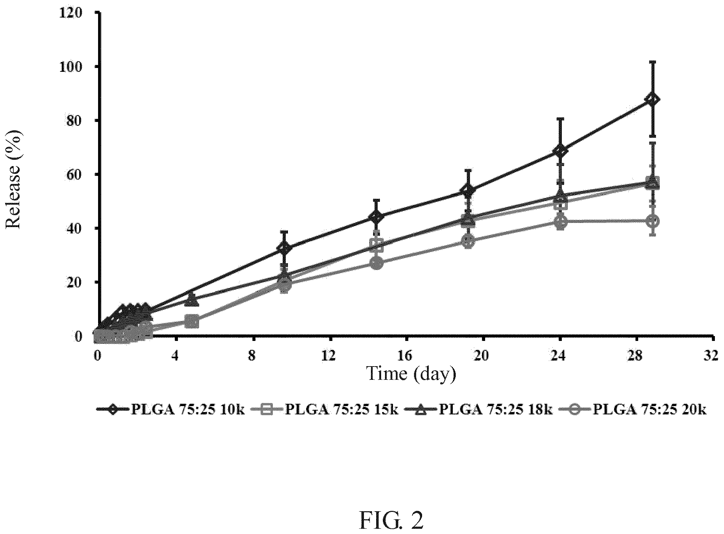

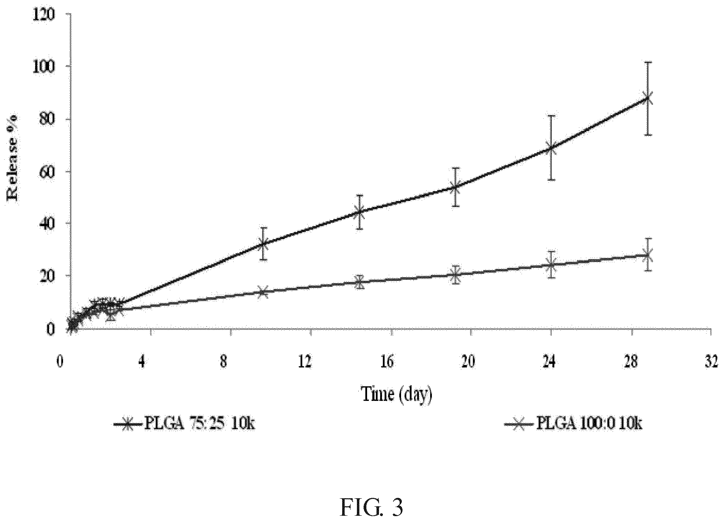

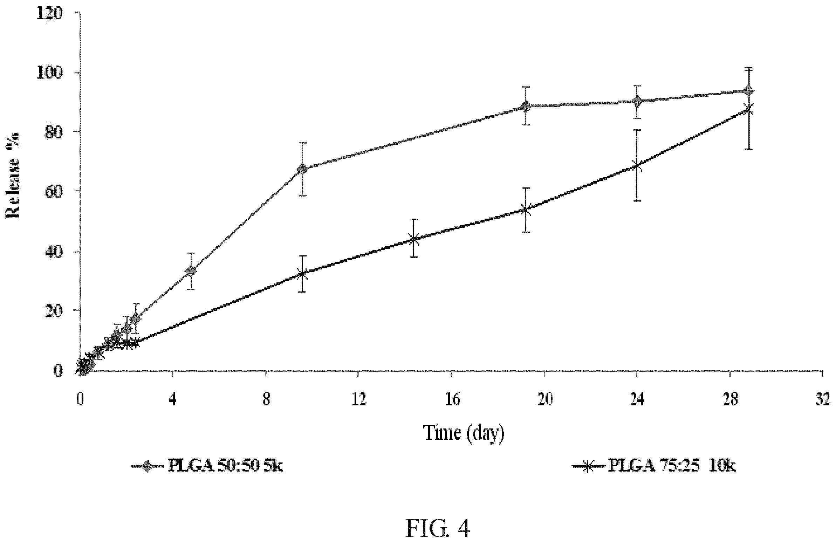

Comparison of the dissolution results of SDE-PLGA microspheres with the same PLA/PGA ratios and different molecular weights including (75/25 10k), (75/25 15k), (75/25 18k), and (75/25 20k) indicated the lower the molecular weight, the higher the drug release rate. The drug release rate of 75/25 10k, 75/25 15k, 75/25 18k, and 75/25 20k at day 30 was 87.71%±13.81%, 56.64%±6.40%, 57.30%±14.33%, and 42.78%±5.42%, respectively (mean±SD are presented, n=3) (Figure 2). Comparison of the dissolution results of SDE-PLGA microspheres with different PLA/PGA ratios and same molecular weights, (75/25 10k) and (100/0 10k), suggested that the lower the PLA concentration, the higher the drug release rate, and the drug release rate of 75/25 10k and 100/0 10k at day 30 was 87.71%±13.81% and 28.16%±6.31%, respectively (mean±SD, n=3) (Figure 3). Moreover, comparison of the dissolution results of SDE-PLGA microspheres with different PLA/PGA ratios and different molecular weights, (50/50 6k) and (75/25 10k), indicated the same results as shown above that the lower the molecular weight and PLA concentration, the higher the drug release rate. The drug release rate of 75/25 10k and 100/0 10k at day 30 was 88.54%±6.47% and 87.71%±13.81%, respectively (mean±SD are presented, n=3) (Figure 4).

**Example 3 In vivo Pharmacokinetic studies of controlled release SDE- PLGA formulation form**

**Experimental design and description:**

The aim of the present study is to determine and compare the absorption, distribution, and elimination of a single dose of different prescription drugs and formulation forms in rats. Analysis of plasma nalbuphine and SDE collected from rats intramuscular injected with a single dose of controlled release SDE-PLGA revealed the information of distribution, metabolism, and elimination of different formulation forms in rats as well as provided the evidence showing the selected formulation is effective for at least a month.

**1. Pharmacokinetics studies of controlled release SDE-PLGA formulation form after intramuscular injection in small animal rats**

**Experiment animals:**

    i. Species: Rat
    ii. Strain: Sprage-Dawley
    iii. Source: National Laboratory Animal Center
    iv. Initial animal age: 6-9 week old young adult mice
    v. Initial animal weight: between 200-300 g, the smaller the differences the better.
    vi. Mark: on the tail
    vii. Group size: 6-7 female mice/group

**Experiment period:** The rats were subjected to observation and the blood samples were collected after administration of the drug until no nalbuphine was detected (about a month).
**Administration routes and methods:** A single dose of long-term controlled release SD-PLGA adjusted according to the body weight was intramuscular injected.
**Analysis of drug formulation:** The prepared drug is further analyzed for its chemical strength as well as for its uniformity and stability which is essential for any formulation.

**Method design:**

    i. Animal adaptation and selection: upon arrival at the lab, around 25 female rats were subjected to one week of adaptation and observation period. During the observation period, the general health condition and signs of disease will be monitored closely. All rats were vaccinated properly by providers and were given a complete exam by the vet prior to initiate the experiment.

ii. Animal selection: Rats showed any disease symptom or abnormal physiological sign were excluded from the study after observation period.

iii. Housing conditions: Rats were housed in a temperature and humidity controlled environment (at 20-30 °C with 30-70% humidity) and with a 12 hr light control.

iv. Animal feed: The food used was Rodent Chow® #5002 purchased from Purina Mills, and the food supply is unlimited and meal discontinuation prior to the experiment is not necessary.

v. Water: Unlimited water supply.

vi. Random selection: Xybion random program was used for random selection of animals and the body weight was used as the grouping standard for random selection to ensure the weights of rats in each group are evenly distributed and each group has at least six female rats.

vii. Drug administration: SDE-PLGA microspheres containing 150 mg/kg SDE was suspended in 1.25% CMC, and was given via intramuscular injection at the right thigh.

viii. Plasma sample collection

Blood samples were collected from the tail vein and the rats were placed in a restraining device to reduce stress induced by drug administration and sample collection. Afterwards, 150 mg per kg of SDE-PLGA microspheres were given to the rats. Blood samples (0.5 ml) were collected at 0.5, 1, 2, 6, 24, 30, 48, 54, 72, 96, 102, 120, 168, 240 hr and 12, 14, 16, 18, 20, 24, 26, 28 days after administration of the drug, and mixed with heparin (1/10, v/v). After centrifugation, the supernatant was transferred and stored at -80 °C. During the experiment, rats exhibited critical conditions were subjected to euthanasia using carbon dioxide.

**Sample analysis: A well-developed Ultra Performance Liquid Chromatography** (UPLC/Ms/Ms) was used to analyze nalbuphine and SDE in collected samples.

**Data analysis:** The serum variations of the administered drugs are presented in the graphs and figures. In addition, the in vivo pharmacokinetics results of nalbuphine and SDE including relative bioavailability in rats, is calculated with software.

## 2. UPLC/MS/MS analysis conditions:

Ultra Performance Liquid Chromatography (UPLC) analysis: a Mass/ Mass UPLC/MS/MS interfaced with a triple quadrupole tandem and equipped with ionspray (API 3000 Applied biosystems, U.S.A.) was used for analysis: The chromatographic separation was achieved using an ACQUITY UPLC/BEH HILIC/2.1 X column (100 mm/1.73 um/40°C). The mobile phase consisted of water (13%) and acetonitrile (87 %) (containing 2 mM ammonium acetate and 0.1% formic acid), was injected at a flow rate of 0.25 ml/min and the injection volume was $5\mu L$. The precursor ion and the product ion used for analyze sebacoyl dinalbuphine ester, nalbuphine, ethyl morphine, and naloxone were 441.5(m/z) and 423.5(m/z), c358.3(m/z) and 340.3(m/z), 314.2(m/z) and 183.1(m/z), and 328.3(m/z) and 310.3 (m/z), respectively.

## 3. Plasma sample processing

i. Internal standards including 50 $\mu$l each of ethyl-morphine (2 $\mu$g/ml) and naloxone (200 ng/ml) were added to the bottom of a 16x125mm test tube.

ii. A fixed volume of 50 $\mu$l $Na_2CO_3$ (0.5 N, pH=10.0) was then added (basic drugs reduced from ionic state to molecular state)

iii. Extraction solution (containing Ether and DCM, 7:3 v/v) 4 ml was added to the tube on ice and stored at 4°C cold room to prevent degradation of the pro-soft drug SDE in plasma.

iv. Collected plasma (100 $\mu$l) was added and vortex for 10 min and immediately centrifuged at 3,000 RPM for 10 min.

v. Incubated at -80 to solidify the lower water phase, and then the supernatant was transferred to a new 13x100 mm test tube.

vi. Vacuum-dried with nitrogen at 10 psi and at 40°C for 20 min (Long time drying may result in degradation of the pro-soft drug).

vii. A sample of diluted solution (200$\mu$l, ACN: water, 85:15) was added to the test tube to resuspend the pro-soft drug and 5$\mu$l were injected into UPLC/MS/MS.

## 4. Preparation of Calibration curves:

The mixtures of pro-soft drug sebacoyl dinalbuphine ester and the prodrug nalbuphine were formulated at concentrations of 10, 25, 50, 100, 250, 500, 1000, 2500, and 5000 ng/ml in standard solvent, ACN. From each mixture,

10 $\mu$l was transferred and mixed with 90 $\mu$l plasma (10X dilution) for further sample processing.

Following UPLC/MS/MS analysis, the peak areas and their corresponding concentrations shown in the chromatography of sebacoyl dinalbuphine ester and nalbuphine were used to plot two calibration curves, and the accuracy and precision of the curves were further examined using standard deviation (SD), coefficient of variation (% CV), and error (%).

**Results:**

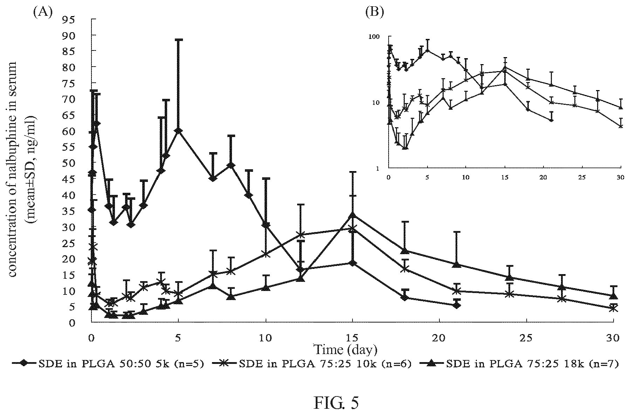

The obtained results indicated that drug concentration can be regulated by adjusting the polymer characteristics (e.g. PLA/PGA ratio and average molecular weight) which is consistent with the previous theory. Moreover, the drug release rate (decreased in the order of 50:50 5k> 75:25 10k> 75:25 18k) can regulate drug concentrations and therefore maintain an effective serum concentration at 2.5 ng/ml for two weeks or up to several months (Figure 5). Likewise, the total released drug amount also decreased in the order of 50:50 5k> 75:25 10k> 75:25 18k. These results further confirmed the theory of using the ratios of PLA/PGA and average molecular weight to regulate the drug concentration in the serum (please refer to Table 3 for various parameters).

**Table 3 In vivo pharmacokinetics parameters of nalbuphine. Administration of the SD rats with long-term controlled release 50:50 5k (150 mg/kg SDE), 75:25 10k (150 mg/kg SDE), and 75:25 18k (150 mg/kg SDE).**

| PK parameter (unit) | SDE-PLGA 50:50 5k IM 150 mg/kg (N=5) | SDE-PLGA 75:25 10k IM 150 mg/kg (N=6) | SDE-PLGA 75:25 18k IM 150 mg/kg (N=7) |
|---|---|---|---|
| k(1/day) | 0.15±0.03 | 0.13±0.03 | 0.09±0.03 |
| AUC0→t(day*ng/ml) | 602.73±61.57 | 433.66±54.87 | 424.36±116.77 |
| AUC0→∞ (day*ng/ml) | 640.58±61.57 | 471.84±51.16 | 530.60±133.87 |
| T1/2(day) | 4.92±0.96 | 5.87±1.72 | 8.52±2.77 |
| Cl/F(L/day/kg) | 237.03±30.73 | 327.06±36.32 | 302.42±95.16 |
| Vd/F(L/kg) | 1673.07±338.97 | 2919.23±948.70 | 3631.51±1218.53 |

In summary, the pro-soft drug mentioned in the invention is a formulation comprising of sebacoyl dinalbuphine ester and a common pharmaceutical and biodegradable excipient, PLGA polymer. The said formulation can be prepared in a controlled release form. Polymer allows the sustained release of the drug ,which subsequently prolongs the effective concentration of nalbuphine in blood and dramatically reduces the frequency of traditional nalbuphine injections.

**Claims**

1. A long-term controlled release formulation form of nalbuphine that significantly reduces the injection dosage, wherein the formulation is comprising of:

   (a) sebacoyl dinalbuphine ester, and
   (b) at least one pharmaceutical acceptable and biodegradable excipient PLGA polymer, wherein the PLGA excipient includes at least one of the following: PLA, PGA, or derivatives or combinations of PLA and PGA, the ratios of PLA/PGA are 50-100 %/0-50% and the range of molecular weight of PLGA is 5 k-20 K.

2. A pharmaceutical formulation form as recited in claim 1, wherein the formulation form includes pharmaceutically acceptable salts, solvents, or relevant derivatives which is pharmaceutically functional for medical treatment.

3. A pharmaceutical formulation form as recited in claim 1, wherein the derivatives of PLA or PGA include poly butylene succinate (PBS), polyhydroxyalkanoate (PHA), polycaprolactone acid lactone (PCL), polyhydroxybutyrate (PHB), glycolic amyl (PHV), PHB and PHV copolymer (PHBV), and poly lactic acid (PLA) - polyethylene glycol (PEG) copolymers (PLEG).

4. A pharmaceutical formulation form as recited in claim 1, wherein the formulation was prepared in one of the following forms: tablets, capsules, soft capsules, granules, suspensions, microspheres, oral implants, implantable injections, emulsion injection, and other pharmaceutically acceptable long-term released formulation forms.

**Patentansprüche**

1. Formulierungsform von Nalbuphin mit kontrollierter Langzeitwirkstofffreisetzung, die die Injektionsdosis signifikant verringert, wobei die Formulierung besteht aus:

   (a) Sebacoyldinalbuphinester, und
   (b) zumindest einem pharmazeutisch unbedenklichen und biologisch abbaubaren Exzipienten PLGA-Polymer, wobei der PLGA-Exzipient zumindest eines des Folgenden beinhaltet: PLA, PGA oder Derivate oder Kombinationen von PLA und PGA, wobei die Verhältnisse von PLA/PGA 50 bis 100 %/0 bis 50 % betragen und der Molekulargewichtsbereich von PLGA 5 k bis 20 K ist.

2. Pharmazeutische Formulierungsform nach Anspruch 1, wobei die Formulierungsform pharmazeutisch unbedenkliche Salze, Lösungsmittel oder relevante Derivate beinhaltet, die für eine medizinische Behandlung pharmazeutisch funktionell ist.

3. Pharmazeutische Formulierungsform nach Anspruch 1, wobei die Derivate von PLA oder PGA Polybutylensuccinat (PBS), Polyhydroxyalkanoat (PHA), Polycaprolactonsäurelacton (PCL), Polyhydroxybutyrat (PHB), Glykolamyl (PHV), Copolymer (PHBV) von PHB und PHV und Copolymere (PLEG) von Polymilchsäure (PLA) und Polyethylenglykol (PEG) beinhalten.

4. Pharmazeutische Formulierungsform nach Anspruch 1, wobei die Formulierung in einer der folgenden Formen hergestellt wurde: Tabletten, Kapseln, Weichkapseln, Granulaten, Suspensionen, Mikrokügelchen, Oralimplantaten, implantierbaren Injektionen, Emulsionsinjektion und anderen pharmazeutisch unbedenklichen Formulierungsformen mit Langzeitwirkstofffreisetzung.

**Revendications**

1. Forme de formulation à libération contrôlée de longue durée de nalbuphine qui réduit de façon significative le dosage par injection, dans laquelle la formulation se compose de :

   (a) ester de sébacoyl dinalbuphine ; et
   (b) au moins un polymère PLGA excipient pharmaceutiquement acceptable et biodégradable, l'excipient PLGA comprenant au moins l'un des suivants : PLA, PGA ou dérivés ou combinaisons de PLA et PGA, les rapports de PLA/PGA étant de 50-100 %/0-50 % et la plage de masse moléculaire de PLGA étant 5 k-20 K.

2. Forme de formulation pharmaceutique selon la revendication 1, dans laquelle la forme de formulation comprend les sels pharmaceutiquement acceptables, les solvants ou les dérivés pertinents, qui est pharmaceutiquement fonctionnelle pour un traitement médical.

3. Forme de formulation pharmaceutique selon la revendication 1, dans laquelle les dérivés de PLA ou PGA comprennent le polybutylène succinate (PBS), un polyhydroxyalcanoate (PHA), la polycaprolactone acide lactone (PCL), un polyhydroxybutyrate (PHB), l'amyle glycolique (PHV), un copolymère PHB et PHV (PHBV) et les copolymères (PLEG) acide polylactique (PLA)-polyéthylène glycol (PEG).

4. Forme de formulation pharmaceutique selon la revendication 1, dans laquelle la formulation a été préparée dans l'une des formes suivantes : comprimés, capsules, capsules molles, granulés, suspensions, microsphères, implants oraux, injections implantables, injection d'émulsion et autres formes de formulation à libération à long terme, pharmaceutiquement acceptables.

FIG.1

FIG. 2

—◆— PLGA 75:25 10k —□— PLGA 75:25 15k —△— PLGA 75:25 18k —○— PLGA 75:25 20k

FIG. 3

PLGA 75:25 10k

PLGA 100:0 10k

Release %

Time (day)

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 085106156 A

- WO 089109293 A